Europäisches Patentamt

European Patent Office

Office européen des brevets

(11) Publication number: **0 089 548**
**B1**

## EUROPEAN PATENT SPECIFICATION

(45) Date of publication of patent specification: **28.01.87**

(21) Application number: **83102265.2**

(22) Date of filing: **08.03.83**

(51) Int. Cl.⁴: **A 61 M 31/00,** A 61 K 9/02,
A 61 K 9/22

(54) Osmotic drug delivery system.

(30) Priority: **18.03.82 US 359447**
**18.03.82 US 359324**

(43) Date of publication of application:
**28.09.83 Bulletin 83/39**

(45) Publication of the grant of the patent:
**28.01.87 Bulletin 87/05**

(84) Designated Contracting States:
**AT BE CH DE FR GB IT LI LU NL SE**

(56) References cited:
**EP-A-0 040 457**
**FR-A-2 386 316**
**US-A-3 732 865**
**US-A-3 797 492**
**US-A-3 948 254**
**US-A-4 210 139**

(73) Proprietor: **MERCK & CO. INC.**
**126, East Lincoln Avenue P.O. Box 2000**
**Rahway New Jersey 07065 (US)**

(72) Inventor: **Higuchi, Takeru**
**2811 Schwarz Road**
**Lawrence Kansas 66044 (US)**
Inventor: **Himmelstein, Kenneth J.**
**1640 Hillcrest**
**Lawrence Kansas 66044 (US)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

Courier Press, Leamington Spa, England.

## Description

The invention relates to an osmotic drug delivery device shaped and sized for oral ingestion, implantation, rectal, vaginal or ocular insertion, for delivery of a drug or other beneficial substance. The device comprises a compartment consisting of two or more chambers in series. The chambers house osmotically active agents, adsorption adjuvants, pro-drugs, enzymes, drugs, pesticides and the like. At least one of the chambers contains an osmotically active agent and at least one chamber contains the drug or other beneficial substance. The chambers are formed by an external shell and one or more chamber-dividing walls. The external shell is made of a rigid microporous material.

Osmotic drug delivery systems make possible the administration of an active agent and a controlled and continuous rate to achieve a predetermined useful effect in animals including humans.

An osmotic drug delivery device is known in accordance with the prior art portion of claim 1 (EP—A—0 040 457) which consists of two chambers and in which the chamber-dividing wall is semipermeable or impermeable to fluid agents and solutes. Part of the exterior of the chamber containing the osmotic agent may be of a semipermeable material. The osmotic pressure in the one chamber decreases the volume of the other chamber containing the drug so that it is dispensed through the microporous shell. Because the osmotic pressure is transmitted to the drug-containing chamber through deformation of the chamber-dividing wall the drug delivery rate is influenced by outside agitation.

US—A—3,845,770 discloses an osmotic drug delivery device in which the drug is dispensed through a minute orifice in the wall of the device. Such a device tends to administer the active agents at a relatively high concentration to a relatively small area of body tissue. Due to the relatively high concentration local toxic reactions may be caused and the absorption of the drug may be decreased. In addition, the orifice through which the active agent is dispensed can be subject to mechanical plugging.

The invention as claimed in claim 1 solves the problem of how to design an osmotic delivery system whose release rate is independent of outside agitation and which avoids local toxic reactions.

The advantages offered by the invention are mainly that the drug is dispensed at a controlled rate for a prolonged period of time, that the device is designed with a minimum number of parts, that a complete pharmaceutical dosage regimen for a particular time period can be administered, the use of which requires intervention only for initiation and termination of the regimen, that a high concentration of an active agent can be contained therein and that the high concentration of the active agent will not exhibit the tendency to be leached from the device nor have its potency decreased by chemical breakdowns. The device may contain a product which can be used as an osmotically effective solute to exhibit an osmotic pressure gradient against an external fluid. The device of the invention releases the drug at a rate which does not vary with time. The device can contain the drugs in various physiochemical forms such as esters, salts and the like that can be heat sterilized by conventional techniques. The release rate can be chosen ranging from very low to very high by using polymeric wall forming materials in combination with the active agent or mixture of an active agent and other osmotically effective compound. The device can be made from erodible or biodegradable materials that erode or degrade after the device has released the active agent.

Co-pending application EP—A—0 180 708 also relates to an osmotic drug delivery device.

Preferred embodiments of the invention are subject matter of the subclaims. Several ways of carrying out the invention are described in detail below with reference to drawings, in which:—

Figure 1 is a cross-section of a device of this invention with two chambers, showing the semipermeable membrane overlay, 1, the microporous rigid shell, 2, the dividing wall, 3, with the orifice, 4, separating chamber 5 containing osmotic agents, and chamber 6 containing the active agent.

Figure 2, also is a cross-section of a device of this invention with three chambers, showing a relatively resistant semi-permeable membrane overlay, 9, a relatively permeable semi-permeable membrane 1, the microporous shell 2, dividing walls, 3 and 8, with orifices, 4, separating chamber 7 containing an absorption adjuvant, chamber 5, containing an osmotic agent, and chamber 6 containing the active agent.

Figure 3 is a cross-section of one embodiment of the device of this invention with two chambers 5, 6 showing the semi-permeable membrane overlayer 1, the microporous rigid shell, 2, the dividing wall, 3, separating chamber 5 containing osmotic agents, and chamber 6 containing the active agent.

Figure 4, also is a cross-section of a device of this invention with three chambers, showing a relatively resistant semi-permeable membrane overlay, 9, a relatively permeable semi-permeable membrane 1, the microporous shell 2, dividing walls, 3 and 8, separating chambers 5, 6 and 7.

The Figures show an osmotic delivery system shaped and sized for oral ingestion, implantation, rectal, vaginal or ocular insertion for delivery of a drug or other beneficial substance comprising a compartment consisting of one or more chambers 5, 6, 7 in series, the chambers being adapted to house osmotically active agents, adsorption adjuvants, drugs, and pro-drugs. The chambers 5, 6, 7 are formed by an external shell and one or more chamber-dividing walls 3, 8, each chamber-dividing wall optionally having a micro-orifice 4 connecting adjacent chambers. The external shell 2 and chamber-dividing walls 3, 8 are of a rigid microporous material. The device comprises one or more successive overlayers 1, 9 of a semipermeable membrane, the first overlayer 1 completely covering the external shell 2 of all but one chamber and substantially covering the external shell of the one chamber 6 leaving exposed a microporous drug-emitting surface. Each successive overlayer 9 completely covers all but one more chamber and substantially covers the one more chamber. The device optionally includes a quick release loading dose of drug external to and covering the microporous drug-emitting surface 10 designed for spontaneous removal following establishment of desired flow characteristics.

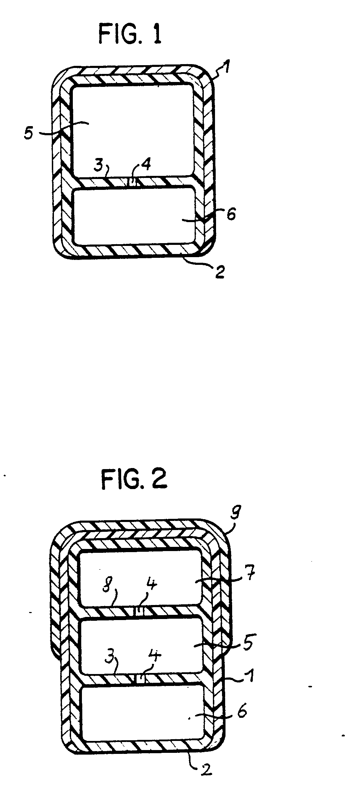

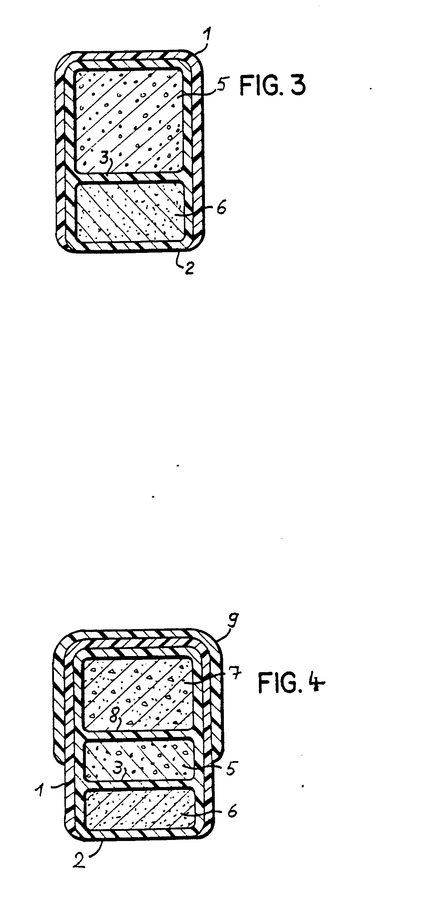

The upper part of the device of this invention with two chambers consists of a chamber (Figures 1 and 3) containing an osmotically active agent which may or may not be a pharmacologically active agent or pro-drug, especially a relatively soluble pro-drug form of a relatively insoluble drug. The shell of that chamber is designed to be relatively inflexible but semi-permeable to water. The semi-permeability to water and suitable rigidity can be obtained, for example, by coating a microporous, rigid shell 2 of a relatively hard, preferably biodegradable polymer, with an appropriate semi-permeable substance or membrane 1. The pores of the shell of the upper chamber may or may not be filled with the osmotically active agent during production.

The lower chamber 6 of the device, which can be formed externally from the same microporous shell 2 as the upper part, but not necessarily is filled with the drug or other beneficial substance to be delivered or an appropriate formulation thereof including, but not necessarily, additional osmotically active agent. The upper and lower chambers 5, 6 are connected and separated by a chamber-dividing wall 3 optionally with a small orifice 4 opening into the two chambers 5, 6. The porosity of the shell 2 of the lower chamber 6 is selected so as to maintain a small positive pressure within the chamber with respect to the exterior during use of the device.

In its operation, the device is introduced to the appropriate site such as the rectum, muscle, gastrointestinal tract, vagina, or cul de sac. Because of the substantial area of the semipermeable membrane 1 exposed by the upper part of the device, water is slowly drawn into the upper chamber 5 from the adjacent tissue by osmotic action, at a rate controlled by the water permeability of the semi-permeable membrane 1. The aqueous solution which is produced in the upper chamber 5 at a more or less constant rate by this process flows through the microporous chamber dividing wall 3 or orifice 4 connecting the two chambers and eventually carries with it the drug substance stored in the lower chamber 6 through the microporous shell 2. The overall rate of delivery is determined by the total osmotic volume flow rate generated across the semi-permeable membrane 1 and the amount of the drug substance transported to the surface of the device per unit volume of the osmotically-produced aqueous fluid.

Although in Figure 1 the orifice connecting the lower and upper chambers is shown to be a simple opening offering no significant resistance to flow, it is possible for the present invention to utilize a controlling valve in its place for special purposes, for example for controlling the manner of delivery other than that of constant flow or zero order delivery.

One special embodiment of this capability is to incorporate a valve in place of the orifice 4 which will not allow any significant flow to take place until a certain preselected pressure develops in the upper chamber 5. At this pressure, the flow will take place until the pressure is substantially relieved. The valve closes at this point and will not open again until the preset pressure is again reached. This intermittent mode of flow will produce pulsed delivery of the drug substance to the surface of the device according to any special requirement. This mode of rectal drug delivery is of special advantage when absorption adjuvants are to be co-administered. Continuous mode of delivery may not present to the absorbing tissue high concentrations simultaneously of both the drug and adjuvant. Pulsed delivery will permit this to take place more effectively. The interval between pulses may be as short as 10 to 20 minutes or as long as several hours.

Another embodiment of the present invention includes a quick-releasing loading dose of the drug substance external to the drug reservoir on the surface of the microporous shell 2 which would eventually serve as the drug-emitting surface. This is a desirable feature since the pumping action will not take place immediately on insertion of the device.

In embodiments of the novel device wherein an appreciable portion of the drug-emitting surface must be exposed it is convenient to employ a cap that will spontaneously be removed when proper flow characteristics are achieved. Spontaneous removal can be the result of achieving some predetermined internal osmotic pressure in which case the cap is composed of a slowly bioerodible material. On the other hand, if the cap can be readily expelled from the body it can be made of quite impervious materials. In another mode of operation the cap is composed of a bioerodible material having dispersed therein a loading dose of the drug or other beneficial substance.

Another embodiment of this invention is that which is specially desirable when adjuvants are to be co-delivered by the device or the active agent is to be delivered slowly by means of dilute solutions. Although the configuration shown in Figures 1 or 3 may often suffice where the adjuvant may be included in the upper chamber 5 and/or the lower chamber 6, in some instances the designs shown in Figure 2 or 4 offer greater flexibility in selection of both the osmotic and adjuvant agents. In this configuration, there are three chambers 5, 6, 7 with the adjuvant being placed in the uppermost chamber 7 which has a semi-permeable external barrier of relatively low permeability. The middle chamber 5 will contain largely appropriate osmotic agent and will present a relatively highly semi-permeable (aqueous) barrier surface to the outside. The bottom chamber 6 still serves as the drug reservoir. This design will permit greater flexibility in selecting both the osmotic agent and the adjuvant. The difference in the mode of operation between the designs shown in Figure 1 or 3 and Figure 2 or 4 is apparent in that with the former if a solid adjuvant is present in the upper chamber serving both as a source of the adjuvant and as the osmotic agent, the adjuvant will surface outside the drug reservoir more or less as a saturated solution. In the second configuration, however, the adjuvant can be delivered in a substantially diluted, subsaturation concentration because of the diluting effect produced by influx of water into the middle chamber 5. It is evident that this will permit greater flexibility in use of the invention for various drugs and various adjuvants and will allow closer approach to optimal temporal pattern for a given drug.

In the configuration represented schematically in Figure 2, pulsed delivery can be effected by placing the valving system between the middle and lower chambers 5, 6.

Although there is no limit to the number of chambers that can be incorporated in the novel device of this invention, for various specialized purposes, it is preferred that it have not more than three and preferably only two chambers.

The substance forming a large part of the outer surface of the device is semi-permeable, for example a material that is permeable to an external fluid such as water and the like while essentially impermeable to a selected product or other compounds in the device. This material can be non-erodible or bioerodible after a predetermined period of time and in each instance it is semi-permeable to solvent but not to solute and is suitable for construction of the outer layer of the device. Typical materials for forming the wall include membranes known to the art as osmosis and reverse osmosis membranes such as commercially available unplasticized cellulose acetate, plasticized cellulose acetate, rein-forcedcellulose acetate, cellulose nitrate with 11 percent nitrogen, cellulose diacetate, cellulose triacetate, agar acetate, amylose triacetate, beta glucan acetate, beta glucan triacetate, cellulose acetate, acetaldehyde dimethyl acetate, cellulose acetate ethyl carbamate, cellulose acetate phthalate, cellulose acetate methyl carbamate, cellulose acetate succinate, cellulose acetate dimethaminoacetate, cellulose acetate ethyl carbonate, cellulose acetate chloroacetate, cellulose acetate ethyl oxalate, cellulose acetate methyl sulfonate, cellulose acetate butyl sulfonate, cellulose acetate proprionate, cellulose acetate p-toluene sulfonate, triacetate or locust gum bean, cellulose acetate with acetylated hydroxyethyl cellulose, hydroxylated ethylene-vinylacetate, cellulose acetate butyrate having a viscosity of from about 10 seconds to about 50 seconds, cellulose acetate butyrate containing about 17 percent of combined butyryl and about 29.5 percent acetyl, perm- selective, aromatic nitrogen-containing polymeric membranes that exhibit water permeability and essentially no solute passage, osmosis membranes made from polymeric epoxides, osmosis membranes made from copolymers of an alkylene oxide and alkyl glycidyl ether, semi-permeable polyurethanes, semi-permeable polyglycolic or polylactic acid and derivatives thereof, thin film membranes as disclosed by Loeb and Sourirajan in US—A—3,133,132, the membranes of ionically associated polyelectrolytes, the polymers formed by the coprecipitation of polycation and a polyanion as described in US—A—3,276,586; 3,541,005; 3,541,006; 3,546,142; 3,173,876; derivatives of polystyrene such as poly(-sodium styrenesulfonate) and poly(vinylbenzyltrimethyl-ammonium chloride), and the like. Generally, membranes, having a fluid permeability of 0.01 to 10 $cc/cm^2/hour$ or day/or higher at atmosphere pressure against a saturated product solution or saturated solute solution to a changing concentration at the temperature of use while simultaneously possessing a high degree of impermeability to the product or solute are useful.

The preferred materials are the cellulose acetates, especially cellulose triacetate.

The microporous material from which rigid shell 2 is composed can be described as having a sponge-like appearance that provides a supporting structure for microscopic-sized interconnected pores or voids. The materials can be isotropic wherein the structure is homogenous throughout a cross-sectional area, or they can be anisotropic wherein the structure is non-homogenous throughout a cross-sectional area. The pores can be continuous pores that have an opening on both faces of microporous material, pores interconnected through tortuous paths of regular and irregular shapes including curved, curved-linear, randomly oriented continuous pores, and other porous paths discernible by microscopic examination. Generally microporous materials are defined by the pore size, the number of pores, the tortuosity of the microporous path and the porosity which relates to the size and the number of pores. The pores size of microporous material is easily ascertained by measuring the observed pore diameter at the surface of the material under the electron microscope. Generally, materials possessing from 5 to 95% pores and having a pore size of from 100 nm (10 angstroms) to about 100 μm (microns) can be used for making the device. The pore size and other parameters characterizing the microporous structure also can be obtained from flow measurements as discussed in US—A—3,977,404.

Microporous materials are commercially available materials and can be made by art known methods. The materials can be made by etched nuclear tracking, by cooling a solution of flowable polymer below the freezing point whereby solvent evaporates from the solution in the form of crystals dispersed in the polymer and then curing the polymer followed by removing the solvent crystals, by cold or hot stretching at low or high temperatures until pores are formed, by leaching from a polymer a soluble component by an appropriate solvent, by ion exchange reaction, and by polyelectrolyte processes. Processes for preparing microporous materials are described in Synthetic Polymer Membranes, by R. E. Kesting, Chapters 4 and 5, 1971, published by McGraw Hill, Inc.; Chemical Reviews, Ultrafiltration. Vol. 18, pages 373 to 455, 1934; Polymer Eng. and Sci., Vol. 11, No. 4, pages 284 to 288, 1971; J. Appl. Poly. Sci., Vol. 15, pages 811 to 829, 1971; and in US—A—3,565,259, 3,615,024; 3,751,536; 3,801,692, 3,852,224; and 3,849,528.

Microporous materials useful for making the devices include microporous polycarbonates comprised of linear polyesters of carbonic acid in which carbonate groups recur in the polymer chain, microporous materials prepared by the phosgenation of a dihydroxyl aromatic such as bisphenol A, poly(vinylchloride), microporous polyamides such as polyhexamethylene adipamide, microporous modacrylic copolymers including those formed of poly(vinylchloride) 60% and acrylonitrite, styrene-acrylic and its copolymers, porous polysulfones characterized by diphenylene sulfone groups in a linear chain thereof, halogenated poly(vinylidene), polychloroethers, acetal polymers, polyesters prepared by esterification of a dicarboxylic acid or anhydride with an alkylene polyol, poly(alkylenesulfides), phenolic polyesters, asymmetric porous polymers, cross-linked olefin polymers, hydrophobic or hydrophilic microporous homopolymers, copolymers or interpolymers having a reduced bulk density, and materials described in US—A—3,595,752; 3,643,178; 3,654,066; 3,709,774; 3,718,532; 3,803,061; 3,852,224; 3,853,601; and 3,852,388, in GB—A—1,126,849, and in Chem. Abst., Vol. 71, 4274f, 22572f, 22573f, 1969.

· Additional microporous materials include, polyolefins, poly(urethanes), cross-linked, chain-extended poly(urethanes), microporous poly(urethanes) in US—A—3,524,753, poly(imides), poly(benzimidazoles), collodion (cellulose nitrate with 11% nitrogen), regenerated proteins, semi-solid cross-linked poly(vinylpyrrolidone), microporous materials prepared by diffusion of multivalent cations into polyelectrolyte sols as in US—A—3,565,259, anisotropic permeable microporous materials of ionically associated polyelectrolytes, porous polymers formed by the coprecipitation of a polycation and a polyanion as described in US—A—3,276,589, 3,541,005, 3,541,006, and 3,546,142, derivatives of poly(styrene) such as poly(sodium styrenesulfonate) and poly(vinyl benzyltrimethylammonium chloride), the microporous materials disclosed in US—A—3,615,024, 3,646,178 and 3,852,224. Other microporous materials include those that slowly erode over time, or erode after the device has released the agent; such as, cross-linked gelatin, cross-linked poly(lactide), cross-linked poly(vinyl alcohol) and poly(glycolide).

The preferred microporous materials are fabricated from the cellulosics described earlier, preferably the cellulose triacetate.

Representative of compositions of matter that can be released from the device and can function as a solute are without limitation those compositions soluble in aqueous type fluids such as tear fluid, tissue juices, water; organic solvents and the like. The expression "composition of matter" as used in this disclosure is meant to include the terms product, active agent, beneficial agent and the like, and these terms are deemed as functionally equivalent for the present invention. These compositions are osmotically effective as solutes since they can generate a solvent concentration gradient between the exterior medium and the medium inside the device. These compositions include organic and inorganic compounds such as ephedrine hydrochloride, ephedrine sulfate, hydroxyamphetamine, isoproterenol hydrochloride, carbachol, pilocarpine hydrochloride, pilocarpine nitrate, demecarium bromide, ecothiophate iodide, physostigmine salicylate, timolol maleate, homatropine hydrochloride, homatropine methylbromide, methscopolamine nitrate, alverine citrate, chlorphenoxamine, hydrochloride, calcium pantothenate and the like. Additional compositions that can be administered are those that produce a physiologically or pharmacologically useful effect at a point in near relation to the delivery device, or compositions that will produce a physiological or pharmacological response at a site remote from the point of release from the device include drugs generically known as, without limitation, hypnotics, sedatives, psychic energizers, tranquilizers, anticonvulsants, muscle relaxants, analgesics, anti-inflammatories, anesthetics, anti-spasmodics, anti-ulcer agents, anti-microbials, hormonal agents, cardiovascular agents, diuretics, neoplastic agents, and the like.

The composition, drug or the like can also be in various forms, such as uncharged molecules, components of molecular complexes, pharmacologically acceptable salts such as hydrochloride, hydrobromide, sulfate, phosphate, nitrate, borate, acetate, maleate, tartrate, salicylate and the like. For acidic drugs, salts of metals, amines, or organic cations, for example quaternary ammonium can be employed. Furthermore, simple derivatives of the drug such as esters, ethers, amide, and the like which have good solubility characteristics are suitable for the purpose of the invention. Also, a product or drug that is water insoluble can be used in a form that is a water soluble derivative thereof to effectively serve as a solute, and on its release from the device is converted by enzymes, hydrolyzed by body pH, or other metabolic processes to the original form or to a biologically active form. Additionally, the drug formulation can have various art known forms such as solution, dispersion, paste, cream, particle, granule, tablet, emulsions, suspensions, powders and the like.

Various osmotically effective solutes including organic and inorganic compounds are advantageously used when it is desired to release a composition, product, drug or the like having limited solubility from the device. The term "limited solubility" as used herein means that the compound has a solubility of less than about 1% by weight in the external fluid, that is, the ratio of the weight of the compound in solution to the weight of the water of that solution is less than 1 percent. The term includes low, slightly and moderate solubility of the composition in the field. The osmotically effective compounds or solutes confined in the device are a substantial motive force of the device and they exhibit an osmotic pressure gradient against an external fluid across the membrane while the membrane is substantially impermeable to the passage of the osmotically effective solute to prevent loss thereof through the membrane. The solutes are conveniently used by dispensing or homogeneously or heterogeneously mixing a solute or a mixture of solutes with the composition, active agent, product or the like either before they are charged into the compartment or by self mixing after charging a solute and composition into the compartment. In operation, these solutes osmotically attract fluid into the device to produce a solution of the solute which is released from the device concomitantly transporting therewith undissolved and dissolved composition, product, drug or the like. Various osmotically effective solutes include compounds such as magnesium sulfate, magnesium chloride, sodium chloride, lithium chloride, potassium sulfate, sodium carbonate, sodium sulfite, lithium sulfate, calcium bicarbonate, sodium sulfate, calcium sulfate, potassium acid phosphate, calcium lactate, magnesium-succinate, tartaric acid, soluble carbohydrates such as raffinose, glucose, mixtures thereof and the like. The solid solute, present initially in excess, can be in any suitable physical form such as particles, crystals, pellets, tablets, strips, film, granules and the like.

The preferred osmotic agents are sodium chloride, sodium carbonate, and calcium bicarbonate.

Additionally, the composition and composition solute can be used in a mixed form by mixing the composition or product with a binder. The product in powdered, granular, piece and the like form, is homogeneously or heterogeneously dispersed in the binder which binder is water soluble or water insoluble but will release the product on contact with water. Typical water soluble binders include polyethylene glycol, gelatin, agar, carboxycellulose, ethylmethylcellulose, polyvinyl alcohol, polyvinylpyrrolidone, water soluble starch derivatives and the like. Typical water insoluble binders that can comprise about 1 to 50 percent of the composition include cellulose acetate, polyurethane, epoxides, and other insoluble binders that permit the free movement of water into the pores of the structure to transport the product from the binder.

The amount of composition present in the device, whether soluble, a derivitized soluble form thereof, is generally non-limited and it is an amount larger than or equal to the amount of the composition that is necessary to osmotically operate the device and on its release from the device is effective for bringing about the product's desired effect. Since the invention contemplates a variety of devices of various sizes and shapes, for a variety of uses, there is no critical upper limit on the amount of product incorporated in the device. The lower limit will depend on osmotic activity, the span of the release of the product and the activity of the product. Generally, the device will contain about 0.01 percent to 90 percent or higher of a product or a mixture of product and solute based on the weight of the product or product solute to the volume of the device, and the like. Typically, the device can be of such size and shape to release 0.01 cc to 5 cc or higher of product contained in the fluid per hour, day or longer, such as 1 cc to 10 cc of product solution for 1 to 10 days, and the like.

The optional micro-orifice in the wall separating the chambers of the novel delivery device of this invention is approximately of the same size as described in US—A—3,845,770 and is about $2 \times 10^{-3}$ to about $8 \times 10^{-3}$ cm in length and about $0.3 \times 10^{-3}$ to about $5 \times 10^{-3}$ cm in diameter.

The technology relating to the fabrication of the novel device of this invention is well known in the pharmaceutical art.

**Claims**

1. An osmotic delivery device shaped and sized for oral ingestion, implantation, rectal, vaginal or ocular insertion, for delivery of a drug or other beneficial substance comprising a compartment consisting of two or more chambers (5, 6, 7) in series, at least one of the chambers (5) containing an osmotically active agent and at least one of the chambers (6) containing the drug or other beneficial substance, the chambers (5, 6, 7) being formed by an external shell (2) and one or more chamber-dividing walls (3), and the external shell (2) being of a rigid microporous material, characterized by

the chamber-dividing walls (3) also being of the rigid microporous material and

one or more successive overlayers of a semipermeable membrane (1), the first overlayer completely covering the external shell (2) of all but one chamber (5) and substantially covering the external shell (2) of the one chamber (5) leaving exposed a microporous drug-emitting surface and each successive overlayer (9) completely covering all but one more chamber and substantially covering the one more chamber (6).

2. The osmotic delivery device of claim 1, wherein each chamber-dividing wall (3) has a micro-orifice (4) connecting adjacent chambers (5, 6, 7).

3. The osmotic delivery device of claim 1 or 2, wherein a quick release loading dose of drug is external to and covers the microporous drug-emitting surface.

4. The osmotic delivery system of claim 1, 2, or 3, wherein the microporous material of the external shell (2) and the semipermeable membranes (1) of the overlayers are fabricated from a cellulose acetate.

5. The osmotic delivery system of claim 4, wherein the micro-orifice (4) connecting the chamber (5) with the drug-emitting surface and the adjacent chamber (6) comprises a pressure actuated controlling valve.

6. The osmotic delivery system of any one of claims 1 to 5 wherein a cap covers the drug-emitting surface and is designed for spontaneous removal following establishment of desired flow characteristics.

## Patentansprüche

1. Eine durch Osmose wirksame Abgabevorrichtung, die in Form und Größe für orale Einnahme, Implantation, rektales, vaginales oder okulares Einlegen bestimmt ist, für die Abgabe eines Arzneimittels oder einer anderen nützlichen Substanz, umfassend eine aus zwei oder mehreren Kammern (5, 6, 7) in Reihe bestehende Abteilung, wobei wenigstens eine der Kammern (5) ein osmotisch wirksames Mittel enthält, und wenigstens eine der Kammern (6) das Arzneimittel oder die andere nützliche Substanz enthält, wobei die Kammern (5, 6, 7) durch ein Außengehäuse (2) und eine oder mehrere Kammerteilungswand bzw. -wände (3) gebildet sind, und das Außengehäuse (2) aus einem steifen, mikroporösen Material besteht, dadurch gekennzeichnet,

daß die Kammerteilungswände (3) auch aus dem steifen, mikroporösen Material und

einer oder mehreren aufeinanderfolgenden Auflagen aus einer semipermeablen Membran (1) bestehen, wobei die erste Auflage das Außengehäuse (2) aller Kammern, außer einer Kammer (5), vollständig bedeckt, und das Außengehäuse (2) der einen Kammer (5) im wesentlichen bedeckt, jedoch eine mikroporöse, Arzneimittel emittierende Oberfläche exponiert läßt, und jede folgende Auflage (9) sämtliche Kammern, außer einer weiteren Kammer, vollständig bedeckt, und die eine weitere Kammer (6) im wesentlichen bedeckt.

2. Die durch Osmose wirksame Abgabevorrichtung des Anspruchs 1, wobei jede Kammerteilungswand (3) eine Mikroöffnung (4) aufweist, die benachbarte Kammern (5, 6, 7) verbindet.

3. Die durch Osmose wirksame Abgabevorrichtung des Anspruchs 1 oder 2, wobei eine rasch freisetzbare Belastungsdosis des Arzneimittels außerhalb der mikroporösen, Arzneimittel emittierenden Oberfläche angeordnet ist und diese bedeckt.

4. Das durch Osmose wirksame Abgabesystem des Anspruchs 1, 2 oder 3, wobei das mikroporöse Material des Außengehäuses (2) und die semi-permeablen Membranen (1) der Auflagen aus einem Celluloseacetat hergestellt sind.

5. Das durch Osmose wirksame Abgabesystem des Anspruchs 4, wobei die Mikroöffnung (4), die die Kammer (5) mit der Arzneimittel emittierenden Oberfläche und der benachbarten Kammer (6) verbindet, ein druckbetätigtes Regelventil enthält.

6. Das durch Osmose wirksame Abgabesystem irgendeines der Ansprüche 1 bis 5, wobei eine Kappe die Arzneimittel emittierende Oberfläche bedeckt und zur spontanen Entfernung nach Einstellung der gewünschten Fließkennmerkmale vorgesehen ist.

## Revendications

1. Dispositif osmotique de libération de forme et de taille adaptées à l'ingestion orale, l'implantation, l'insertion rectale, vaginale ou oculaire, pour la libération d'un médicament ou autre substance bénéfique comprenant un compartiment constitué de deux ou plusieurs chambres (5, 6, 7) en série, au moins une des chambres (5) contenant un agent osmotiquement actif et au moins une des chambres (6) contenant le médicament ou autre substance bénéfique, les chambres (5, 6, 7) étant formées par une enveloppe externe (2) et une ou plusieurs parois divisant les chambres (3), et l'enveloppe externe (2) étant d'une matière microporeuse rigide, caractérisé en ce que

les parois divisant les chambres (3) sont également faites de la matière microporeuse rigide et

un ou plusieurs recouvrements successifs, d'une membrane semi-perméable (1), le premier recouvrement recouvrant complètement l'enveloppe externe (2) de toutes les chambres sauf une (5) et recouvrant essentiellement l'enveloppe externe (2) de ladite chambre (5) laissant exposée une surface microporeuse émettrice de médicament et chaque recouvrement successif (9) recouvrant complètement toutes les chambres sauf une autre chambre et recouvrant essentiellement cette autre chambre (6).

2. Dispositif de libération osmotique de la revendication 1, où chaque paroi divisant les chambres (3) a un micro-orifice (4) reliant les chambres adjacentes (5, 6, 7).

3. Dispositif de libération osmotique des revendications 1 ou 2, où une dose de charge de libération rapide de médicament est externe à et recouvre la surface microporeuse émettrice de médicament.

4. Système de libération osmotique des revendications 1, 2 ou 3 où la matière microporeuse de l'enveloppe externe (2) et les membranes semiperméables (1) des recouvrements sont fabriquées à partir d'un acétate de cellulose.

5. Système de libération osmotique de la revendication 4, où le microorifice (4) reliant la chambre (5) avec la surface émettrice de médicament et la chambre adjacente (6) comprend une soupape de régulation actionnée par la pression.

6. Système de libération osmotique d'une quelconque des revendications 1 à 5 où un capuchon recouvre la surface émettrice de médicament et est conçu pour un enlèvement spontané après l'établissement des caractéristiques d'écoulement désirées.

## FIG. 1

## FIG. 2

FIG. 3

FIG. 4